# EUROPEAN PATENT SPECIFICATION

(11) **EP 1 660 123 B1**
(45) Date of publication and mention of the grant of the patent: **13.03.2013**
(21) Application number: 04764688.0
(22) Date of filing: 31.08.2004
(51) Int. Cl.: A61K 39/15, C12N 7/00

(54) **ROTAVIRUS VACCINE**
ROTAVIRUS-VAKZINE
VACCIN ROTAVIRUS

(30) Priority: 02.09.2003 US 499430 P; 01.07.2004 GB 0414787
(43) Date of publication of application: 31.05.2006
(60) Divisional application: 10176849.7
(73) Proprietor: GlaxoSmithKline Biologicals s.a., 1330 Rixensart (BE)
(72) Inventor: COLAU, Brigitte Desiree Alberte, B-1330 Rixensart (BE); DE VOS, Beatrice Arsene Virginie, B-1330 Rixensart (BE)
(74) Representative: Crepin, Carine Marie Blanche
(86) International application number: PCT/EP2004/009725
(87) International publication number: WO 2005/021033

(56) References cited:
- WO-A2-01/12797
- US-A- 5 474 773
- BARNES G L ET AL: "Early phase II trial of human rotavirus vaccine candidate RV3" VACCINE, BUTTERWORTH SCIENTIFIC. GUILDFORD, GB, vol. 20, no. 23-24, 26 July 2002 (2002-07-26), pages 2950-2956, XP004371823 ISSN: 0264-410X
- PEREZ-SCHAEL I. et al.: 42nd Interscience Conference on Antimicrobial Agents and Chemotherapy (ICAAC 2002), 27-30 September 2002, San Diego: "Protective efficacy of an oral human rotavirus (HRV) vaccine in latin american infants". XP002318589 cited in the application
- BERNSTEIN D I ET AL: "Safety and immunogenicity of live, attenuated human rotavirus vaccine 89-12", VACCINE, ELSEVIER LTD, GB, vol. 16, no. 4, 1 February 1998 (1998-02-01), pages 381-387, XP004099298, ISSN: 0264-410X, DOI: 10.1016/S0264-410X(97)00210-7

## Description

### FIELD OF THE INVENTION

The invention relates to the use of an attenuated rotavirus population from one rotavirus serotype in the prevention of disease associated with rotavirus infection from another rotavirus serotype. In particular the invention relates to the use of an attenuated rotavirus population from G1 serotype in the prevention of disease associated with rotavirus infection from non-G1 serotypes.

### BACKGROUND

Acute, infectious diarrhoea is a leading cause of disease and death in many areas of the world. In developing countries, the impact of diarrhoeal disease is staggering. For Asia, Africa and Latin America, it has been estimated that there are between 3-4 billion cases of diarrhoea each year and of those cases about 5-10 million result in death (Walsh, J.A. et al.: N. Engl. J. Med., :301:967-974 (1979)).

Rotaviruses have been recognised as one of the most important causes of severe diarrhoea in infants and young children (Estes, M.K. Rotaviruses and Their Replication in Fields Virology, Third Edition, edited by Fields et al., Raven Publishers, Philadelphia, 1996). It is estimated that rotavirus disease is responsible for over one million deaths annually. Rotavirus-induced illness most commonly affects children between 6 and 24 months of age, and the peak prevalence of the disease generally occurs during the cooler months in temperate climates, and year-round in tropical areas. Rotaviruses are typically transmitted from person to person by the faecal-oral route with an incubation period of from about 1 to about 3 days. Unlike infection in the 6-month to 24-month age group, neonates are generally asymptomatic or have only mild disease. In contrast to the severe disease normally encountered in young children, most adults are protected as a result of previous rotavirus infection so most adult infections are mild or asymptomatic (Offit, P.A. et al. Comp. Ther., 8(8):21-26, 1982).

Rotaviruses are generally spherical, and their name is derived from their distinctive outer and inner or double-shelled capsid structure. Typically, the double-shelled capsid structure of a rotavirus surrounds an inner protein shell or core that contains the genome. The genome of a rotavirus is composed of 11 segments of double-stranded RNA which encode at least 11 distinct viral proteins. Two of these viral proteins designated as VP4 and VP7 are arranged on the exterior of the double-shelled capsid structure. The inner capsid of the rotavirus presents one protein, which is the rotavirus protein designated VP6. The relative importance of these three particular rotaviral proteins in eliciting the immune response that follows rotavirus infection is not yet clear. Nevertheless, the VP6 protein determines the group and subgroup antigen, and VP4 and VP7 proteins are the determinants of serotype specificity.

To date, at least 14 rotavirus G serotypes and 11 rotavirus P serotypes have been identified (Linhares A.C. & Bresse J.S., Pan. Am. J. Publ. Health 2000, 9, 305-330). Among these, 10 G serotypes and 6 P serotypes have been identified among the human rotavirus.

VP7 protein is a 38,000 MW glycoprotein (34,000 MW when non-glycosylated) which is the translational.product of genomic segment 7, 8 or 9, depending on the strain. This protein stimulates formation of the neutralising antibody following rotavirus infection. VP4 protein is a non-glycosylated protein of approximately 88,000 MW which is the translational product of genomic segment 4. This protein also stimulates neutralising antibody following rotavirus infection.

Since VP4 and VP7 proteins are the viral proteins against which neutralising antibodies are directed, they are believed to be prime candidates for development of rotavirus vaccines, affording protection against rotavirus illness.

Natural rotavirus infection during early childhood is known to elicit protective immunity. A live attenuated rotavirus vaccine is thus highly desirable. Preferably this should be an oral vaccine, as this is the natural route of infection of the virus.

Early vaccine development for preventing rotavirus infections began in the 1970s after the discovery of the virus. Initially, attenuated strains from animals and humans were studied and had mixed or disappointing results. More recent efforts have focused on human-animal reassortants that have been more successful.

A rotavirus strain known as 89-12 has been described by Ward; see US 5474773 and Bernstein, D.L. et al, Vaccine, 16 (4), 381-387, 1998. The 89-12 strain was isolated from a stool specimen collected from a 14 month-old child with natural rotavirus illness in 1988. According to US 5474773 the HRV 89-12 human rotavirus was then culture-adapted by 2 passages in primary African Green Monkey Kidney (AGMK) cells and 4 passages in MA-104 cells as described by Ward in J. Clin. Microbiol., 19, 748-753, 1984. It was then plaque purified 3 times in MA-104 cells (to passage 9) and grown after 2 additional passages in these cells. One additional passage was made (passage 12) for deposition with the ATCC under the accession number ATCC VR 2272. The deposited strain is known as 89-12C2.

The 1998 paper in Vaccine by Bernstein et al is referred to below as the Vaccine (1998) paper. The paper describes the safety and immunogenicity of an orally administered live human rotavirus vaccine candidate. This vaccine was obtained from strain 89-12, attenuated by passaging without plaque purification 26 times in primary AGMK cells and then another 7 times in an established AGMK cell line (33 passages in total).

Hereinafter the aforesaid material which has been serially passaged 26 times will be referred to as P26 and the material which has been serially passaged 33 times will be referred to as P33. In general, rotavirus derived by passaging 89-12 n times will be referred to as Pn.

In the examples which follow the P33 material was passaged a further 5 times on Vero cells. This is referred to as P38.

The P26 and P33 isolates described in the Vaccine (1998) paper were not deposited in a culture collection, nor were they analysed to establish their genetic characterisation.

It has been found that the P26 population described in the literature comprises a mixture of variants. This has been established by genetic characterisation as described hereinbelow (see examples). P26 is therefore not a reliably consistent population for further passages, in particular for the production of vaccine lots. Similarly, P33 comprises a mixture of variants and is not reliably consistent for the production of vaccine lots.

It has been found that the P26 material is a mixture of at least three VP4 gene variants. P33 and P38 are similarly a mixture of two variants. These variants appear to be antigenically different, in terms of neutralising epitopes, to the 89-12C2 strain deposited at the ATCC when evaluating the neutralizing antibody titers of sera from infants vaccinated with P33 against these variants.

Furthermore it has been found that when the P33 material is administered to infants, two identified variants are replicated and excreted. Of 100 vaccinated infants, only 2 showed signs of gastro-enteritis due to rotavirus infection, while 20% of a placebo group were infected. These findings suggest that the identified variants are associated with protection from rotavirus disease.

WO 0112797 discloses a method of separating rotavirus variants and an improved live attenuated rotavirus vaccine derived from a cloned (homogeneous) human rotavirus. strain. Also disclosed is an attenuated rotavirus population (isolate), characterised in that it comprises a single variant or substantially a single variant, said variant defined by the nucleotide sequence encoding at least one of the major viral proteins designated as VP4 and VP7. Protective efficacy of such an oral attenuated human rotavirus vaccine against G9 heterologous strain has been reported in Latin American infants (Perez et al. 42nd Interscience Conference on Antimicrobial Agents and Chemotherapy (ICAAC 2002) 27-30 September 2002, San Diego).

### BRIEF DESCRIPTION OF THE FIGURES

Figure 1 is the nucleotide sequence encoding the VP4 protein of P43.
Figure 2 is the nucleotide sequence encoding the VP7 protein of P43.

### DETAILED DESCRIPTION OF THE INVENTION

The invention in its broadest sense is as detailed in the independent claims.

In one aspect of the invention, there is provided a use of an attenuated human rotavirus strain from a G1 serotype in the preparation of a composition for inducing an immune response against rotavirus infection from the G1 and at least two of the non-G1 rotavirus serotypes selected from the group consisting of: G2, G3, G4 and G9 serotypes, wherein the attenuated rotavirus strain is a single rotavirus variant, or substantially a single rotavirus variant, having a VP4 gene comprising, in the nucleotide sequence, an adenine (A) at positions 788 and 802 and a thymine (T) at position 501 from the start codon; and wherein the VP7 gene comprises, in the nucleotide sequence, a thymine (T) at position 605 and an adenine (A) at position 897 from the start codon.

In another aspect of the invention, there is provided a use of an attenuated rotavirus strain from a G1 serotype in the preparation of a composition for inducing an immune response against rotavirus infection from the G1 and at least two of the non-G1 rotavirus serotypes selected from the group consisting of: G2, G3, G4 and G9 serotypes, wherein the attenuated rotavirus strain is ECACC deposit 99081301, or a single rotavirus variant, or substantially a single rotavirus variant, obtainable or derivable from ECACC deposit 99081301 and having a VP4 gene comprising, in the nucleotide sequence, an adenine (A) at positions 788 and 802 and a thymine (T) at position 501 from the start codon, and wherein the VP7 gene comprises, in the nucleotide sequence, a thymine (T) at position 605 and an adenine (A) at position 897 from the start codon.

In the present disclosure we have determined that an attenuated rotavirus population, characterised in that it comprises a single variant or substantially a single variant, said variant defined by a nucleotide sequence encoding at least one of the major viral proteins designated as VP4 and VP7, can be used as a vaccine to provide cross protection against disease caused by rotavirus infection of a different serotype to that used in the vaccine.

In particular the disclosure describes that a G1 rotavirus population, [for example as deposited at the European Collection of Animal Cell Cultures (ECACC), Vaccine Research and Production Laboratory, Public Health Laboratory Service, Centre for Applied Microbiology and Research, Porton Down, Salisbury, Wiltshire, SP4 0JG, United Kingdom on 13 August 1999 under the deposition number 99081301, under the terms of the Budapest Treaty], can be used to prevent disease caused by both G1 and at least one non-G1 rotavirus serotype, such as the G2, G3, G4 and G9 rotavirus serotypes.

Accordingly the present disclosure relates to use of an attenuated rotavirus population from one rotavirus serotype in the prevention of disease associated with rotavirus infection from another rotavirus serotype, wherein the serotype is defined by reference to the sequence of the rotavirus G protein.

In one aspect, the disclosure describes a rotavirus population characterised in that it comprises a single variant or substantially a single variant, said variant defined by a nucleotide sequence encoding at least one of the major viral proteins designated as VP4 and VP7.

In another aspect, the disclosure describes a rotavirus population for use to provide a cross protective effect comprising VP4 and/or VP7 viral proteins from ECACC deposit 99081301.

In particular, a G1 attenuated rotavirus population made of a single rotavirus variant, or substantially a single rotavirus variant, having a VP4 gene comprising, in the nucleotide sequence, an adenine (A) at positions 788 and 802 and a thymine (T) at position 501 from the start codon; and a VP7 gene comprises, in the nucleotide sequence, a thymine (T) at position 605 and an adenine (A) at position 897 from the start codon [for example as deposited at the European Collection of Animal Cell Cultures (ECACC), Vaccine Research and Production Laboratory, Public Health Laboratory Service, Centre for Applied Microbiology and Research, Porton Down, Salisbury, Wiltshire, SP4 0JG, United Kingdom on 13 August 1999 under the deposition number 99081301, under the terms of the Budapest Treaty], can be used to prevent disease caused by G1 and at least two, more preferably at least three, most preferably at least four non-G1 rotavirus serotypes selected from the group consisting of: G2, G3, G4 and G9.

In particular according to the present disclosure, there is described the use of an attenuated rotavirus strain from a G1 serotype in the manufacture of a vaccine composite for the induction of an immune response against a rotavirus which is not G1 or G9. Further disclosed is an immune response additionally induced against. rotavirus infection by a G9 serotype and/or G1 serotype. Further disclosed is an immune response induced against two or more rotavirus serotypes, these serotypes not being G1 or G9. Typically any serotype other than G1 or G9 are selected from the list consisting of: G2, G3, G4, G5, G6, G7, G8, G10, G11, G12, G13 and G14. It is preferred to have heterotypic protection against at least two, at least three, at least four, more preferably at least five or more of these serotypes different to that of the vaccine composition. Most preferably an immune response is induced against all of the following non-G1 serotypes: G2, G3, G4 and G9.

The disclosure also relates to a method of inducing an immune response against rotavirus infection from a rotavirus serotype, the method comprising administering to a subject a composition comprising an attenuated rotavirus vaccine from a different serotype.

In one aspect, the disclosure describes a method of inducing an immune response against rotavirus non- G9 serotype, the method comprising administering to a subject a composition comprising a rotavirus G1 serotype vaccine comprising a single variant or substantially a single variant as defined herein.

The rotavirus population within the vaccine composition for use according to the invention may be of G1P1A P[8] specificity. Disclosure therein is a rotavirus population comprising VP4 and/or VP7 viral proteins from ECACC deposit 99081301 which is suitable to elicit an immune response and typically, provide a cross protective effect. According to one embodiment of the invention, the rotavirus vaccine, for use is the ECACC deposit 99081301, or is as defined in the claims and derived from that deposit.

In another aspect, the disclosure excludes the use of the rotavirus strain corresponding to ECACC 99081301, in a method or use as indicated above for inducing an immune response to the G9 serotype alone, although use of such a G1 strain for the induction of immune responses to G9 in combination with induction of immune responses to other G serotypes is generally not excluded.

The disclosure relates to G1P8 rotavirus strains in methods or uses as described above.

Preferably the vaccine is for administration in a 2 dose regime.

In one embodiment, the vaccine for use according to the invention provides cross protection against gastro-enteritis. Accordingly, in another aspect there is provided a use according to the invention wherein the composition is up to 60% protective, in a population of vaccinated individuals, against diarrhoea caused by infection of rotaviruses of at least two non-G1 serotypes. In another aspect, there is provided a use according to the invention wherein the composition is up to 81% protective against gastro-enteritis. In a still further aspect, there is provided a use according to the invention wherein the composition comprises a G1 rotavirus strain which is up to 83% protective in a population of vaccinated individuals against severe gastro-enteritis.

Preferably the protection rate against diarrhoea and/or gastro-enteritis and/or severe gastro-enteritis achieved in a population of vaccinated individuals infected by a rotavirus of a different type to that of the attenuated rotavirus present in the composition, is between 10 to 90%, more preferably between 20 to 80%, most preferably at least 50%.

The rotavirus vaccine for use according to the invention to give cross-protection has the following preferred features:

Preferably the rotavirus population for use according to the invention is a cloned variant as defined in the claims.

By a population comprising a single variant, or substantially a single variant, is meant a rotavirus population which does not contain more than 10%, and preferably less than 5% and most preferably less than 1% of a different variant or variants. Virus populations can be purified to homogeneity or substantial homogeneity by passaging on suitable cell types or by performing a series of one or more cloning steps.

An advantage of the invention is that a population comprising a single variant is more suitable for the formulation of a consistent vaccine lot. Particular variants defined by nucleotide sequences encoding the major viral proteins may also be associated with enhanced efficacy in the prevention of rotavirus infection.

The single or substantial single variant in the rotavirus population for use according to the invention the invention is a variant in which the VP4 gene comprises a nucleotide sequence comprising the following: an adenine base (A) at position 788, an adenine base (A) at position 802 and a thymine base (T) at position 501 from the start codon, and in which the VP7 gene comprises a nucleotide sequence comprising a thymine (T) at position 605 and an adenine (A) at position 897.

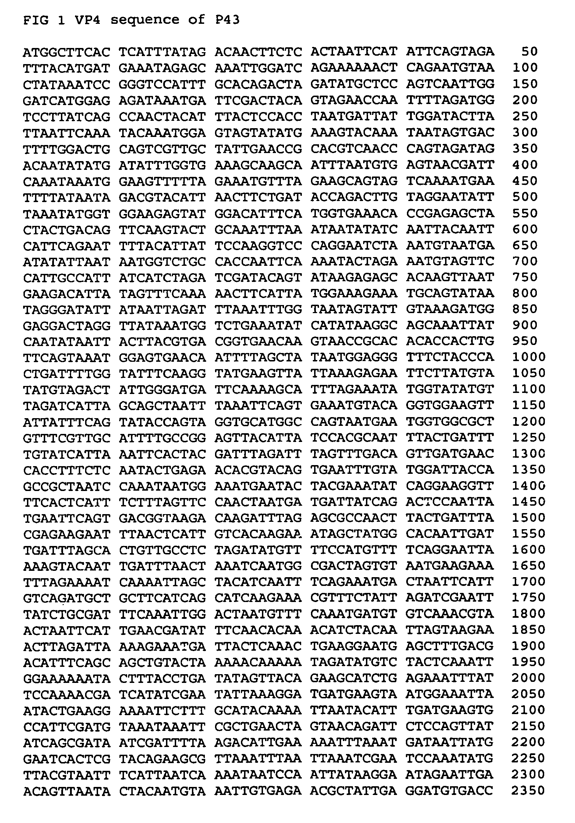

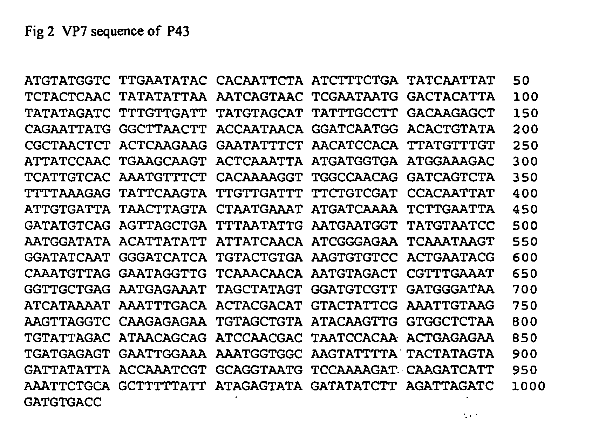

In another aspect, the disclosure describes a single variant comprising a nucleotide sequence encoding a VP4 protein wherein the nucleotide sequence is as shown in Figure 1, and/or a nucleotide sequence encoding a VP7 protein wherein the nucleotide sequence is as shown in Figure 2.

Rotavirus populations for use in the present invention may be obtained by a method comprising:
passaging a rotavirus preparation on a suitable cell type;
optionally selecting homogeneous culture using the steps of either:
   a) limit dilution; or
   b) Individual plaque isolation; and
checking for the presence of a substantially single variant by carrying out a sequence determination of an appropriate region of the VP4 and/or VP7 gene sequence.

Preferably the population is derived from the P33 or P26 strains as described above.

The sequence determination may suitably be carried out by a quantitative or semiquantitative hybridisation technique such as slot blot hybridisation or plaque hybridisation.

Preferably the selected variant is a variant which is replicated and excreted when the starting rotavirus preparation is administered to a human subject, in particular a child.

The resulting cloned virus population resulting from the method described above may be amplified by further passaging on a suitable cell line.

Suitable cell types for passaging the rotavirus population in the above method include African green monkey kidney (AGMK) cells, which may be established cell lines or primary AGMK cells. Suitable AGMK cell lines include for example Vero (ATCC CCL-81), DBS-FRhL-2 (ATCC CL-160), BSC-1 (ECACC 85011422) and CV-1 (ATCC CCL-70). Also suitable are MA-104 (rhesus monkey) and MRC-5 (human - ATCC CCL-171 ) cell lines. Vero cells are particularly preferred for amplification purposes. Passaging on Vero cells gives a high virus yield.

Techniques for checking whether there is a single variant in a virus population resulting from the above method, and for determining the nature of that single variant involve standard sequencing or hybridisation procedures known in the art and are described hereinbelow.

In one aspect, the disclosure describes a method for preparing a rotavirus population suitable for use according to the invention, using a rotavirus having the characteristics of the 89-12 strain or of a passaged derivative thereof.

A single variant population for use according to the invention is P43, which was obtained from P33 (an isolated human rotavirus passages 33 times in culture on appropriate cell types) by a series of end dilution cloning steps followed by passaging the cloned material on Vero cells for amplification.

A P43 population was deposited at the European Collection of Animal Cell Cultures (ECACC), Vaccine Research and Production Laboratory, Public Health Laboratory Service, Centre for Applied Microbiology and Research, Porton Down, Salisbury, Wiltshire, SP4 0JG, United Kingdom on 13 August 1999 under the deposition number 99081301, under the terms of the Budapest Treaty.

Although this indicated public availability is the simplest method of obtaining the human rotavirus P43, similar and functionally substantially identical rotaviruses may be produced by these or other methods in view of the teachings of this disclosure. Such functionally substantially identical rotaviruses are considered to be biologically equivalent to the human rotavirus P43 described therein and therefore their use according to the invention is within the general scope of the present invention. It will therefore be understood that the disclosure encompasses rotavirus populations having the characteristics of the P43 variant as described herein.

It will also be understood that the disclosure encompasses materials derived from the deposited P43 ECACC 99081301 by subjecting it to further processing such as by propagating it by further passaging, cloning, or other procedures using the live virus or by modifying P43 in any way including by genetic engineering techniques or reassortant techniques. Such steps and techniques are well known in the art.

Materials derived from the deposited P43 which are described in the disclosure include protein and genetic material. Of particular interest are reassortant rotaviruses which comprise at least one antigen or at least one segment of P43, for example reassortants which comprise a virulent strain of rotavirus in which one or part of one of the 11 genome segments has been replaced by the genome segment or part thereof of P43. Specifically, a rotavirus reassortant in which the segment or partial segment coding for NSP4 is a P43 segment or partial segment, may have useful properties. Reassortant rotaviruses and techniques for preparing them are well known (Foster, R. H. and Wagstaff, A. J. Tetravalent Rotavirus Vaccine, a review. ADIS drug evaluation, BioDrugs, Gev, 9 (2),155-178, 1998).

Materials of particular interest are progeny of P43 and immunologically active derivatives of P43. Immunologically active derivatives means materials obtained from or with the P43 virus, particularly antigens of the virus, which are capable of eliciting an immune response that is reactive against Rotavirus when injected into a host animal.

In adapting the rotavirus to an appropriate cell line, for example Vero cells, it may be necessary to treat the virus so as to get rid of any potential contaminant such as any adventitious agents that may be present and which would otherwise cause contamination. In the case of ether-sensitive adventitious viruses, this may be done by ether treatment as described hereinbelow. The present disclosure also relates to inclusion of such ether treatment as an optional step in the overall procedure for obtaining an attenuated live rotavirus or vaccine formulated therewith.

The cross protective rotavirus strain for use according to the present invention may be combined with other rotavirus strains to provide additional protection or cross-protection against rotavirus infection or disease.

Therefore, also within the scope of the disclosure are the uses of admixtures of P43 with other rotavirus variants, for example other cloned variants, or with other viruses in particular other attenuated viruses. Such mixtures are useful in the vaccines for use according to the invention which are described hereinbelow.

The present disclosure also describes live attenuated rotavirus vaccine capable of providing cross protection, which comprises a substantially single variant population, admixed with a suitable adjuvant or a pharmaceutical carrier.

Disclosed therein is a rotavirus vaccine being a monovalent rotavirus vaccine containing a single rotavirus strain.

The present disclosure describes a particularly advantageous live rotavirus vaccine in which the live attenuated rotavirus is a human rotavirus and does not cause intussusception.

Suitable pharmaceutical carriers for use with the attenuated rotavirus strain used according to the invention include those known in the art as being suitable for oral administration, especially to infants. Such carriers include and are not limited to carbohydrates, polyalcohols, amino acids, aluminium hydroxide, magnesium hydroxide, hydroxyapatite, talc, titanium oxide, iron hydroxide, magnesium stearate, carboxymethylcellulose, hydroxypropylmethylcellulose, microcrystalline cellulose, gelatin, vegetal peptone, xanthane, caraghenane, arabic gum; ß-cyclodextrin.

The invention also discloses a process for preparing a rotavirus vaccine, for example by freeze drying the virus in the presence of suitable stabilisers or admixing the virus with a suitable adjuvant or pharmaceutical carrier.

It may also be advantageous to formulate the virus for use according to the invention in lipid-based vehicles such as virosomes or liposomes, in oil in water emulsions or with carrier particles. Alternatively or in addition immunostimulants such as those known in the art for oral vaccines may be included in the formulation. Such immunostimulants include bacterial toxins, particularly cholera toxin (CT) in the form of the holotoxin (entire molecule) or the B chain only (CTB) and the heat labile enterotoxin of *E*. *coli* (LT). Mutated LTs (mLTs) which are less likely to convert to their active form than the native LT are described in WO 96/06627, WO 93/13202 and US 5,182,109.

Further immunostimulants which may advantageously be included are saponin derivatives such as QS21 and monophosphoryl lipid A, in particular 3-de-O-acylated monophosphoryl lipid A (3D-MPL). Purified saponins as oral adjuvants are described in WO 98/56415. Saponins and monophosphoryl lipid A may be employed separately or in combination (e.g. WO 94/00153) and may be formulated in adjuvant systems together with other agents. 3D-MPL is a well-known adjuvant manufactured by Ribi Immunochem, Montana and its manufacture is described in GB 2122204.

A general discussion of vehicles and adjuvants for oral immunisation can be found in Vaccine Design, The Subunit and Adjuvant Approach, edited by Powell and Newman, Plenum Press, New York, 1995.

The invention also discloses a method for vaccinating human subjects, especially infants, by administering to a subject in need thereof an effective amount of a vaccine composition for use according to the invention. Preferably the live attenuated vaccine is formulated for oral administration.

In one aspect, the disclosure describes an attenuated rotavirus strain for use according to the invention formulated with an antacid to minimise inactivation of the vaccine by acid in the stomach. Suitable antacid components include inorganic antacids for example aluminium hydroxide AI(OH)₃ and magnesium hydroxide Mg(OH)₂. Commercially available antacids which are suitable for use in the invention include Mylanta (trade mark) which contains aluminium hydroxide and magnesium hydroxide. These are insoluble in water and are given in suspension.

Aluminium hydroxide is a particularly preferred component of a vaccine composition for use according to the invention as it can provide not only an antacid effect but also an adjuvantation effect.

Also suitable for use as antacids in the vaccine for use according to the invention are organic antacids such as organic acid carboxylate salts. A preferred antacid in the vaccine composition for use according to the invention contains an organic acid carboxylate salt, preferably a salt of citric acid such as sodium citrate or potassium citrate.

A particularly preferred antacid that may be used in the vaccine composition for use according to the present invention is the insoluble inorganic salt, calcium carbonate (CaCO₃) The calcium carbonate is able to associate with the rotavirus and the rotavirus activity is maintained during the association with the calcium carbonate.

To prevent sedimentation of calcium carbonate during the filling step, viscous agents are preferably present in the formulation.

Possible viscous agents that may be used include pseudoplastic excipients. A pseudoplastic solution is defined as a solution having higher viscosity on standing compared to its viscosity under agitation. Excipients of this type are natural polymers such as arabic gum, adragante gum, agar-agar, alginates, pectines or semi-synthetic polymers for example: carboxymethylcellulose (Tyloses C®), methylcellulose (Methocels A®, Viscotrans MC®, Tylose MH® and MB®), hydroxypropylcellulose (Klucels®), and hydroxypropylmethylcellulose (Methocels E® and K®, Viscontrans MPHC®). In general those pseudoplastic excipients are used together with thixotropic agents. Alternative viscous agents that may be used are pseudoplastic excipients with low flowing capacity. Those polymers, at a sufficient concentration, give rise to a structural fluid arrangement resulting in a high viscosity solution having low flowing capacity on standing. A certain quantity of energy needs to be given to the system to allow flowing and transfer. External energies (agitation) are needed to destroy temporarily the structural fluid arrangement in order to obtain a fluid solution.
Examples of such polymers are Carbopols® and xanthane gum.

Thixotropic excipents become a gel structure on standing whilst under agitation they form a fluid solution. Examples of thixotropic excipients are: Veegum ®(Magnesium-aluminium silicate) and Avicel RC® (about 89% microcrystalline cellulose and 11 % Carboxymethylcellulose Na).

The vaccine composition for use according to the present invention preferably comprises a viscous agent selected from xanthane gum or starch.

Thus the vaccine composition for use according to the present invention may be formulated with a combination of calcium carbonate and xanthane gum.

Other components of a composition for use according to the invention suitably include sugars for example sucrose and/or lactose.

The vaccine composition for use according to the invention may contain additional components including for example flavourings (particularly for an oral vaccine) and bacteriostatic agents.

Different presentations of the vaccine composition for use according to the invention are envisaged.

In one aspect, the disclosure describes a vaccine for use according to the invention which is administered as a liquid formulation. Preferably the liquid formulation is reconstituted prior to administration from at least the following two components:
i) virus component
ii) liquid component.

In this embodiment, the virus component and the liquid component are normally present in separate containers, which may conveniently be separate compartments of a single vessel, or separate vessels which can be connected in such a way that the final vaccine composition is reconstituted without exposing it to the air.

Prior to reconstitution, the virus may be in a dry form or a liquid form. Preferably the virus component is lyophilised. Lyophilised virus is more stable than virus in an aqueous solution. The lyophilised virus may be suitably reconstituted using a liquid antacid composition to produce a liquid vaccine formulation. Alternatively the lyophilised virus may be reconstituted with water or aqueous solution, in which case the lyophilised virus composition preferably contains an antacid component.

The vaccine formulation for use according to the invention may comprise a virus component formulated with calcium carbonate and xanthane gum in one compartment or vessel and this is reconstituted with water or aqueous solution present in the second compartment or vessel.

In another aspect, the disclosure describes a vaccine composition for use according to the is a solid formulation, preferably a lyophilised cake which is suitable for immediate when placed in the mouth. Lyophilised formulations may conveniently be provided in the form of tablets in a pharmaceutical blister pack.

In another aspect the disclosure describes a rotavirus vaccine for use according to the invention in the form of a quick dissolving tablet for oral administration.

In another aspect the disclosure describes a composition for use according to the invention comprising a live attenuated rotavirus strain, in particular a human rotavirus strain, wherein the composition is a lyophilised solid capable of immediate dissolution when placed in the mouth.

The quick dissolving tablet for use according to the invention may be dissolved in the mouth of the subject sufficiently quickly to prevent swallowing of the undissolved tablet. This approach is particularly advantageous for paediatric rotavirus vaccines.

The virus for use according to the invention is a live attenuated human rotavirus which may be formulated with an inorganic antacid such as calcium carbonate and a viscous agent such as xanthane gum.

In a further aspect, the present disclosure describes a lyophilised formulation wherein the virus component is any rotavirus strain which is formulated with calcium carbonate and xanthane gum.

Vaccines for use according to the invention may be formulated and administered by known techniques, using a suitable amount of live virus to provide effective protection against rotavirus infection without significant adverse side effects in typical vaccinees. A suitable amount of live virus will normally be between 10⁴ and 10⁷ focus forming units (ffu) per dose. A typical dose of vaccine may comprise 10⁵-10⁶ffu per dose and may be given in several doses over a period of time, for example in two doses given with a two-month interval. Benefits may however be obtained by having more than 2 doses, for example a 3 or 4 dose regimen, particularly in developing countries. The interval between doses may be more or less than two months long. An optimal amount of live virus for a single dose or for a multiple dose regimen, and optimal timing for the doses, can be ascertained by standard studies involving observation of antibody titres and other responses in subjects.

The vaccine for use according to the invention may also comprise other suitable live viruses for protection against other diseases, for example poliovirus. Alternatively other suitable live virus vaccines for oral administration may be given in a separate dose but on the same occasion as the rotavirus vaccine composition for the use according to the invention.

Sera from twelve 4 to 6 month old infants vaccinated with the P33 material as described in the Vaccine (1998) paper were tested for neutralization of P33, P38, P43 and 89-12C2.

The range of neutralization titers of all the tested sera is similar for P33, P38 and P43. The statistical analysis shows no significant difference in the overall neutralization titers against all three viruses. This suggests that the conformational and non-conformational neutralization epitopes of P33, P38 and P43 are equally well recognized by the anti-P33 sera of P33 vaccinated infants. This observation indirectly suggests that the neutralization epitopes revealed in this in vitro assay were not altered between P33, P38 and P43.

The range of neutralization titers of P89-12C2 however significantly differs from P33, P38 and P43. This observation suggests that the conformational and non-conformational neutralization epitopes of P33, P38 and P43 are not equally well recognized by the anti-P33 sera of P33 vaccinated infants. This observation indirectly suggests that the neutralization epitopes revealed in this in vitro assay were altered between 89-12 C2 and P33, P38 and P43.

The present disclosure also describes:
1. The use of an attenuated rotavirus strain from a G1 serotype in the manufacture of a vaccine composition for the induction of an immune response against a rotavirus which is not G1 or G9, wherein the attenuated rotavirus strain is A single rotavirus variant, or substantially a single rotavirus variant having a VP4 gene comprising, in the nucleotide sequence, an adenine (A) at positions 788 and 802 and a thymine (T) at position 501 from the start codon; and wherein the VP7 gene comprises, in the nucleotide sequence, a thymine (T) at position 605 and an adenine (A) at position 897 from the start codon.
2. The use according to 1 wherein an immune response is additionally induced against rotavirus infection by a G9 serotype and/or G1 serotype.
3. The use according to 1 or 2 wherein the immune response is induced against two or more rotavirus serotypes, these serotypes not being G1 or G9.
4. The use as claimed in any of 1 to 3 wherein the serotype other than G1 or G9-serotype is selected from the list consisting of: G2, G3, G4, G5, G6, G7, G8, G10, G11, G12, G13 and G14.

The following, non-limiting, examples illustrate the invention.

### EXAMPLES

### BACKGROUND EXAMPLES 1.9

### Example 1: Demonstration that strain 89-12 at passage 26 (P26) is a mixture of variants

### Sequencing of VP4 and VP7 genes from different passage lots.

Sequencing of VP4 and VP7 genes from passage P26 (primary AGMK cells), passage P33 (established (as opposed to primary) AGMK cell line), passage P41 and passage P43 was performed. Total RNA extraction was reverse transcribed and amplified through PCR in one tube/one step.

Primers Rota 5bis and Rota 29bis amplified the entire VP4 gene and primers Rota 1 and Rota 2bis amplified the entire VP7 gene. The PCR material has been sequenced using different primers (see Table 1).

The passage P26 sequence differed from the passage P33 sequence by 3 bases (at positions 501, 788 and 802 bp from the start codon) in VP4 and by three bases in VP7 (108, 605 and 897 bp from the start codon).

The passage P26 sequence scans of VP4 and VP7 show at mutated positions the presence of the passage P33 sequence as a background. Thus it can be seen that passage P26 is a mixture of at least 2 variants.

The passage P33 sequence scans seem homogenous in VP4 and heterogeneous for VP7 (see Table 2).

Passage P38 (derived from passage 33) was passaged 5 times on Vero cells and displayed the same set of VP4 and VP7 sequences as passage P33 (AGMK cell line). Thus there was no major change in populations between P33 and P38.

**TABLE 1: Oligonucleotides used for RT-PCR and sequencing**

| | name | sequence | position |
|---|---|---|---|
| VP7 | Rota 1 | GGC TTT AAA AGA GAG AAT TTC CGT CTG G | -49 to -22 |
| | Rota 1 bis | GGT TAG CTC CTT TTA ATG TAT GGT A | -16 to 10 |
| | Rota 2bis | GGT CAC ATC GAA CAA TTC TAA TCT AAG | 1014-988 |
| | Rota 7 | CAA GTA CTC AAA TCA ATG ATG G | 266-287 |
| | Rota 12 | TGT TGA TTT TTC TGT CGA TCC AC | 372-394 |
| | Rota 46 | GGT TGC TGA GAA TGA GAA ATT AGC TAT AGT GG | 651-682 |
| | Rota 18 | CCA CTA TAG CTA ATT TCT CAT TCT CAG CAA CC | 682-651 |
| VP4 | Rota 5 | TGG CTT CGC CAT TTT ATA GAC A | 2-23 |
| | Rota 6 | ATT TCG GAC CAT TTA TAA CC | 878-859 |
| | Rota 5bis | TGG CTT CAC TCA TTT ATA GAC A | 2-23 |
| | Rota 6bis | ATT TCA GAC CAT TTA TAA CCT AG | 878-856 |
| | Rota 25 | GGA GTA GTA TAT GAA AGT ACA AAT AAT AG | 268-296 |
| | Rota 26 | CTA TTA TTT GTA CTT TCA TAT ACT ACT CC | 296-268 |
| | Rota 27bis | TCG ATA CAG TAT AAG AGA GCA CAA G | 721-745 |
| | Rota 28 | | |
| | Rota 31 | TTC ATT AAC TTG TGC TCT CTT ATA CTG | 753-727 |
| | Rota 32 | GTA TAT GTA GAC TAT TGG GAT G | 1048-1070 |
| | Rota 45 | CAT CCC AAT AGT CTA CAT ATA C | 1070-1048 |
| | Rota 53 | TGT AAC TCC GGC AAA ATG CAA CG | 1205-1227 |
| | Rota 54 | CGT TGC ATT TTG CCG GAG TTA CA | 1227-1205 |
| | Rota 55 | GTA AGA CAA GAT TTA GAG CGC CA | 1465-1487 |
| | Rota 40 | TGG CGC TCT AAA TCT TGT CTT AC | 1487-1465 |
| | Rota 39 | CTT GAT GCT GAT GAA GCA GCA TCT G | 1703-1727 |
| | Rota 33 | CAG ATG CTG CTT CAT CAG CAT CAA G | 1727-1703 |
| | Rota 34 | CGA TCA TAT CGA ATA TTA AAG GAT G | 2008-2032 |
| | Rota 29bis | CAT CCT TTA ATA TTC GAT ATG ATC G | 2032-2008 |
| | | AGC GTT CAC ACA ATT TAC ATT GTA G | 2335-2311 |

**TABLE 2: oligonucleotides used in hybridization**

| | name | sequence | position |
|---|---|---|---|
| VP7 | Rota 41 | AGT ATT TTA TAC TAT AGT AGA TTA TAT TAA TC | 882-913 |
| | Rota 42 | AGT ATT TTA TAC TAT GGT AGA TTA TAT TAA TC | 882-913 |
| VP4 | Rota 15 | ATC CCC ATT ATA CTG CAT TCC TTT C | 807-783 |
| | Rota 16 | ATC CCT ATT ATA CTG CAT TTC TTT C | 807-783 |
| | Rota 35 | ATC CCC ATT ATA CTG CAT TTC TTT C | 807-783 |
| | Rota 36 | ATC CCT ATT ATA CTG CAT TCC TTT C | 807-783 |

| | | | |
|---|---|---|---|
| The bases shown in bold type in Table 2 are the sites of specific sequence variation in VP4 and VP7. | | | |

**TABLE 3: sequence variation of VP4 and VP7 genes**

| 3.1 | | | | | | |
|---|---|---|---|---|---|---|
| VP4 | | | | VP7 | | |
| | 501 bp 167 aa | 788 bp 263 aa | 802 bp 268 aa | 108 bp 36 aa | 605 bp 202 aa | 897 bp 299 aa |
| P26 (AGMK) | A | G/A | G/A | A | C/T | A |
| P33 (AGMK) | T | A | A | G /A | T/C | A/G |
| P38 (VERO) | T | A | A | A/G | T | G/A |
| P43 (VERO) | T | A | A | A | T | A |

| | | | | | | |
|---|---|---|---|---|---|---|
| N.B. In a second clone from the 3 clones which were developed to the level of production lot, the VP7 897 bp position nucleotide is G, rather than A as in the P43 selected clone. This results in a methionine in place of an isoleucine in the amino acid sequence. Variants corresponding to both the selected P43 clone and the clone in which there is a G in VP7 at 897 bp from the start codon, were excreted in the stools of infants who had been vaccinated with the P33 material. | | | | | | |

In Table 3.1, where there are two alternative bases at a particular position, the first of the two represents the base which appears in a major population and the second is the base which appears in a minor population. Major and minor variant populations are judged by the strength of the signal in sequencing.

| 3.2 | | | | | | |
|---|---|---|---|---|---|---|
| VP4 | | | | VP7 | | |
| | 501 bp 167 aa | 788 bp 263 aa | 802 bp 268 aa | 108 bp 36 aa | 605 bp 202 aa | 897 bp 299 aa |
| P26 (AGMK) | Leu | Gly/Glu | Gly/Arg | Arg | Thr/Met | Ile |
| P33 (AGMK) | Phe | Glu | Arg | Arg/Arg | Met/Thr | Ile/Met |
| P38 (VERO) | Phe | Glu | Arg | Arg/Arg | Met | Met/Ile |
| P43 (VERO) | Phe | Glu | Arg | Arg | Met | Ile |

Table 3.2 shows the amino acid changes resulting from the nucleotide differences between the variants.

**TABLE 4**

| | VP4 ( 788 -802 positions ) | | | | VP7 ( 897 position ) | |
|---|---|---|---|---|---|---|
| | G-G | A-A | A-G | G-A | A | G |
| Probes | Rota 15 | Rota 16 | Rota 35 | Rota 36 | Rota 41 | Rota 42 |
| Passages | | | | | | |
| P26 | - | + | + | + | nd | nd |
| P33 | - | + | - | - | + + | + |
| P38 | - | + | - | - | + | ++ |
| P43 | - | + | - | - | + | - |

### Slot blot hybridization

The change in populations between passages P26 to P33 on AGMK cells has been further confirmed by slot blot hybridization. The VP4 and the VP7 gene fragments generated by RT/PCR were hybridized with oligonucleotide probes specific for each variant (see Table 3.1 and 3.2). In contrast to P26 which hybridized with Rota 16, Rota 35 and Rota 36 and not with Rota 15, the VP4 PCR fragment of the P33 material, at positions 788 and 802 hybridized only with Rota 16 and not with either Rota 15 or Rota 35 or Rota 36. These results established the presence of at least 3 variants in P26 (see Table 4).

For the VP7 PCR fragment of the P33 material, position 897 hybridized with Rota 41 and Rota 42. These results established the presence of at least two variants in the P33 material.

### Example 2: Isolation and characterization of the P43 clone

To isolate P33 components as a homogeneous virus population, three end-point dilutions of P33/AGMK on Vero cells were performed and the resulting virus was used to infect Vero cells.

Positive wells were selected using two criteria: growth demonstrated by the largest number of foci detected in the wells and the most isolated positive wells on the plates, as is done classically. After 3 end dilution passages in 96 well microtiter plates, 10 positive wells were amplified successively on Vero cells and evaluated for their yield.

Based on yield, three clones were developed to passage level of production lot.
Immunorecognition by polyclonal antibodies was shown to be similar both between the three clones and between the clones and P33. Homogeneity of the clones was assessed by slot blot hybridization. The final selection of a single clone was based on yield and sequence.

The selected clone was amplified by successive passages on Vero cells to generate a Master seed, a Working seed and finally production lots.

The selected clone was genetically characterized at different passage levels by sequencing of VP4 and VP7 (identity) and by specific slot blot hybridization of the VP4 and VP7 (homogeneity) of the PCR amplified materials. The sequence of the VP4 and VP7 genes of the P43 material are given in Figures 1 and 2 respectively and are identical to P41.

Homogeneity of the selected clone was assessed by a selective hybridization using oligonucleotide probes discriminating nucleotide changes in VP4 and/or VP7 regions for each variant identified during sequencing of P26/primary AGMK (see Table 4).

The VP4 fragment hybridized with Rota 16 and not with Rota 15, Rota 35 or Rota 36. The VP7 fragment hybridized with Rota 41 and not with Rota 42.

These results confirmed that P43 is a homogeneous population.

### Example 3: Removal of potential adventitious virus

Ether was added to P33 (AGMK grown) to a final concentration of 20% for 1 hr. Ether was then bubbled out with N₂ for 35 min. No impact on the titre of P33 seed was observed.

### Example 4: Formulation of a live attenuated vaccine

The production lots described above are formulated for oral adminstration to infants by the following method.

### 1. Lyophilised virus

Standard techniques are used for preparing virus doses. Frozen purified viral bulk is thawed and diluted with appropriate medium composition, in this case Dulbecco's modified eagle Medium, up to a desired standard viral concentration, in this case 10^{6.2} ffu/ml. The diluted virus is then further diluted with lyophilisation stabiliser (sucrose 4%, dextran 8%, sorbitol 6%, amino-acid 4%) up to the target viral titre, in this case 10^{5.6} ffu/dose. 0.5 ml aliquots of stabilised virus composition are aseptically transferred to 3 ml vials. Each vial is then partially closed with a rubber stopper, the sample is freeze dried under a vacuum, the vial is then fully closed and an aluminium cap is crimped in place around the vial to keep the stopper in place.

For use, the virus is reconstituted using one of the following antacid reconstituents:

### (a) Citrate reconstituent

Sodium citrate is dissolved in water, sterilized by filtration and aseptically transferred into reconstituent containers in 1.5 ml amounts at a concentration of 544 mg Na₃Citrate.2H₂O per 1.5ml dose. The reconstituent containers may be for example 3 ml vials, or 4 ml vials, or 2 ml syringes, or soft plastic squeezable capsules for oral administration. As an alternative to maintaining sterile components under sterile conditions, the final container can be autoclaved.

### (b) Al(OH)₃ reconstituent

An aseptic aluminium hydroxide suspension (Mylanta -trademark) is aseptically diluted in sterile water, aseptically transferred to reconstituent containers (for example 2 ml syringes, or soft plastic squeezable capsules) in 2 ml amounts each containing 48 mg Al(OH)₃. An alternative to using sterile components under sterile conditions is to γ irradiate the aluminium hydroxide suspension (preferably at a diluted stage).

Standard ingredients are included to prevent the suspension from settling. Such standard ingredients include for example magnesium stearate, carboxymethylcellulose, hydroxypropylmethylcellulose, microcrystalline cellulose, and silicone polymers. Bacteriostatic agents for example butylparaben, propylparaben or other standard bacteriostatic agents used in food, and flavourings, may also be included.

### 2. Lyophilised virus with Al(OH)₃ in liquid formulation

Standard techniques are used for preparing virus doses. Frozen purified viral bulk is thawed and diluted with appropriate medium composition, in this case Dulbecco's modified eagle Medium, up to a desired standard viral concentration, in this case 10^{6.2} ffu/ml. Aluminium hydroxide suspension is added to reach a final quantity of 48 mg/dose and the virus composition is diluted with lyophilisation stabiliser (sucrose 4%, dextran 8%, sorbitol 6%, amino-acid 4%) up to the target viral titre, in this case 10^{5.6} ffu/dose. 0.5 ml aliquots of stabilised virus composition are aseptically transferred to 3 ml vials. Lyophilisation and closing of the vials is then carried out as described in part 1.

### 3. Lyophilised virus with Al(OH)₃ for blister presentation

Standard techniques are used for preparing virus doses. Frozen purified viral bulk is thawed and diluted with appropriate medium composition, in this case Dulbecco's modified eagle Medium, up to a desired standard viral concentration, in this case 10^{6.2} ffu/ml. Aluminium hydroxide suspension is added to reach a final quantity of 48 mg/dose and the virus composition is diluted with lyophilisation stabiliser which may be sucrose, dextran or amino-acid 4%, or gelatin, or vegetal peptone, or xanthane up to the target viral titre of 10^{5.6}ffu/dose. An aseptic filling operation is employed to transfer doses of 0.5 ml or preferably less to blister cavities. The composition is lyophilised, and the blister cavities are sealed by thermic sealing.

Optionally standard ingredients are included to prevent the aluminium hydroxide suspension from settling. Such standard ingredients include for example magnesium stearate, carboxymethylcellulose, hydroxypropylmethylcellulose, microcrystalline cellulose, and silicone polymers. Flavourings may also be included.

### Example 5: Rotavirus viral titration for various formulations

### 5.1 :Comparison between lactose and sucrose based formulations:

**Table 5**

| Batch n° | Fomulation composition | Viral titer before lyophilisation | Viral titer after lyophilisation and 1 week at 37°C |
|---|---|---|---|
| 98G06/01 | Lactose:2%; Dextran:4%; Sorbitol:3%; AminoAcids:2% | 10^{5.22} | 10^{4.67} |
| 98G06/03 | Sucrose:2%; Dextran:4%; Sorbitol:3%; AminoAcids:2% | 10^{5.28} | 10^{4.92} |

P43 rotavirus was formulated either with sucrose or with lactose as shown in the table above.
Viral titration before lyophilisation is the viral titre in the completed formulated liquid (containing sucrose dextran sorbitol aminoacids) and without the lyophilisation step.

Good results are those in which a <0.5 log decrease at the lyophilisation step and <0.5 log decrease during the "1 week at 37°C" (accelerated stability test) are achieved. The precision of the viral titration is around + or - 0.2 log.

The results indicate that sucrose may be used instead of lactose.

### 5.2: Effect of arginine and replacement of sorbitol by maltitol:

**Table 6**

| Batch n° | Fomulation composition | Viral titer at time = zero after lyophilisation | Viral titer after lyopjhilisation and 1 week at 37°C |
|---|---|---|---|
| 98L16/01 | Lactose:2%; Dextran:4%; Sorbitol:3%; AminoAcids:2% | 10^{4.8} | 10^{4.8} |
| 98L16/02 | Lactose:2%; Dextran:4%; Sorbitol:3%; AminoAcids:2% Arginine: 3% | 10^{4.8} | 10^{4.9} |
| 98L16/04 | Lactose:2%; Dextran:4%; Maltitol:3%; AminoAcids:2% Arginine: 3% | 10^{4.7} | 10⁵ |

The results demonstrate that the addition of arginine (which is known to improve the stability of the virus during lyophilisation and also provides a basic medium in order to compensate for the stomach acidity) maintains the viral titer.

Sorbitol tends to decrease the glass transition temperature of the lyophilised cake by too great a degree. This can be overcome by using maltitol instead of sorbitol as shown above and the viral titer is still maintained.

### 5.3: Various formulation compositions

This experiment demonstrates that a number of formulations are possible.

**Table 7**

| Batch n° | Fomulation composition | Viral titer before lyophilisation | Viral titer after lyophilisation and 1 week at 37° |
|---|---|---|---|
| 99C11/01 | Sucrose:2%; Dextran:4%; Sorbitol:3%; AminoAcids:2% | 10^{5.24} | 10^{5.07} |
| 99C11/02 | Sucrose:2%; Dextran:4%; Maltitol:3%; AminoAcids:2% | 10^{5.09} | 10^{4.92} |
| 99C11/04 | Dextran:4%; Maltitol:3%; AminoAcids:2% | 10^{4.89} | 10^{5.06} |
| | | | |

| Batch n° | Fomulation composition | Viral titer at time = zero after lyophilisation | Viral titer after lyophilisation and 1 week at 37° |
|---|---|---|---|
| 99C17/01 | Sucrose:2%; Dextran:4%; Sorbitol:3%; AminoAcids:2% | 10^{5.40} | 10^{5.41} |
| 99C17/02 | Sucrose:2%; Dextran:4%; Sorbitol:1.5%; AminoAcids:2% | 10^{5.30} | 10^{4.93} |
| 99C17/03 | Sucrose:2%; Dextran:4%; AminoAcids:2% | 10^{5.31} | 10^{5.24} |
| 99C17/04 | Sucrose:2%; Dextran:4%; Maltitol:3%; AminoAcids:2% | 10^{4.42} | 10^{4.45} |
| 99C17/05 | Sucrose:2%; Dextran:4%; | 10^{4.39} | 10^{4.40} |
| | Maltitol:1.5%; AminoAcids:2% | | |
| 99C17/06 | Sucrose:2%; Dextran:4%; Sorbitol:3%; | 10^{5.44} | 10^{4.97} |
| 99C17/07 | Sucrose:2%; Dextran:4%; Sorbitol:1.5%; | 10^{5.11} | 10^{4.89} |

### 5.4: Association between Rotavirus and Al(OH)₃ antacid:

**Table 8**

| Rotavirus | Al(OH)₃ | H₂O | Contact time at room temperature | Centrifugati on | Supernatant viral titer in ffu/ml | Pellets viral titer in ffu/ml |
|---|---|---|---|---|---|---|
| 10 ^{5.6} ffu/ml | 48 mg in 0.240ml | 0.76 ml | 30 min | 8000rpm, 10 min | 10^{3.66} | |
| 10 ^{5.6} ffu/ml | 0.48 mg in 0.240ml | 0.76 ml | 30 min | 8000rpm, 10 min | 10^{4.41} | |
| 10 ^{5.6} ffu/ml | | 1 ml | 30 min | 8000rpm, 10 min | 10^{5.68} | |
| Rotavirus in Lyophilised Cake | 12 mg in 0.120ml | 1.38 0ml | 30 min | 8000rpm, 10 min | Below detection | 10^{4.7} |

Al(OH)₃ is used as an antacid. This shows that Rotavirus is associated with the insoluble inorganic salt (Al(OH)₃) since it centrifuged together with the Al(OH)₃ (decrease of viral activity in the supernatant).

### 5.5: Dissolution of Al(OH)₃ antacid by SodiumCitrate before viral titration

**Table 9**

| Viral samples | Dissolution | Conditions | Viral titers ffu/ml |
|---|---|---|---|
| 99B10/06 liquid formulation before lyophilisation; 10^{5.43} | 1.5ml Na₃Citrate | 24h at room temperature | 10^{5.11} |
| 99B10/06:lyophilized 10^{5.43} | 1.5ml Na₃Citrate | 24h at room temperature | 10^{4.53} |

When Rotavirus is associated with the Al(OH)₃, it is possible to lyophilise everything (including the Al(OH)₃). After lyophilisation, it is possible to recover the Rotavirus by dissolving Al(OH)₃ in SodiumCitrate. This step does not damage the Rotavirus and retains its activity after this dissolution step.

### 5.6: Infectivity of Rotavirus after liberation of the Al(OH)₃-Rotavirus association:

The mechanism of virus liberation (by dissolution of the carrier) may very well occur *in vivo.* Indeed below pH 6, aluminium hydroxide becomes completely soluble, and thus, Rotavirus will be liberated in the stomach.

Al(OH)₃ + 3 H⁺ → Al⁺⁺⁺ (water soluble) + 3 H₂O

In the stomach, Al⁺⁺⁺ ions are not absorbed (J.J. Powell, R.Jugdaohsingh and R.P.H. Thompson, The regulation of mineral adsorption in the gastrointestinal track, Proceedings of the Nutrition Society (1999), 58, 147-153).
In the intestine, due to the increase of pH, insoluble forms of aluminium are precipitated (Al(OH)₃ or AlPO₄), and eliminated by the natural way.
It is unknown whether the newly formed Al(OH)₃ (or AlPO₄) precipitate will be able to reassociate with free Rotavirus. This raises the question of the infectivity of the Al(OH)₃-Rotavirus association itself.

Liberation of Rotavirus from the Al(OH)₃-Rotavirus association by other mechanisms is also possible. Lysine, for example, interferes with the viral adsorption on Al(OH)₃.
Other anions like borate, sulfate, carbonate and phosphate are known to be specifically adsorbed on aluminium hydroxide, thus, theoretically, it should be possible to displace (by competition for the adsorption site) Rotavirus from the Al(OH)₃-Rotavirus association.

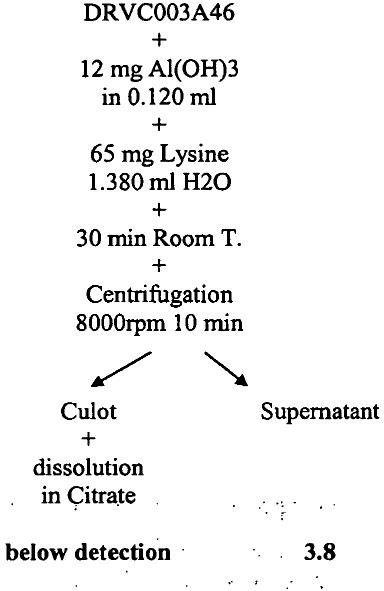

Thus, Rotavirus may be liberated from the Rotavirus - Al(OH)₃ association and the liberated Rotavirus remains active.
This liberation can be done either by dissolving Al(OH)₃ (by HCl in the stomach, or by Na₃Citrate in vitro) or by displacing Rotavirus by a basic amino acid (lysine). **5.7: Infectivity of the Al(OH)₃-Rotavirus association**

A single dose of lyophilised Rotavirus was reconstituted with water and divided into two parts. The first part, considered as the reference, received an additional volume of water. The second part received 24mg of Al(OH)₃ suspended in 0.240 ml of water (Preclinical viral titrations).

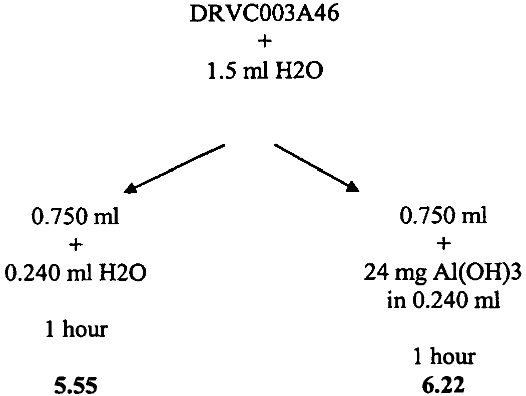

When Al(OH)₃ is present, Rotavirus is active and the viral titration value is higher compared to the reference sample.

This experiment was repeated without dividing the lyophilised dose, and by adding 12 mg Al(OH)₃ or 24 mg Al(OH)₃.

Here the reference sample was the one reconstituted with a Citrate-Bicarbonate buffer. Thus, the viral titer is again higher in the presence of Al(OH)₃.

| DRVC003A46 + 1.5 ml WL buffer | DRVC003A46 + 12 mg Al(OH)3 in 0.120ml + 1.380ml H2O | DRVC003A46 + 24 mg Al(OH)3 in 0.240ml + 1.260ml H2O |
|---|---|---|
| | | |
| **5.34** | **6.24** | **6.05** |
| **5.32** | **5.95** | **6.26** |

As in the example above, Rotavirus associates with the Al(OH)₃ particles, since the virus can be discarded by centrifugation. DRVC003A46 is a lyophilised formulated Rotavirus (Sucrose:2%; Dextran: 4%,Sorbitol:3%; Amino-acids:2%). 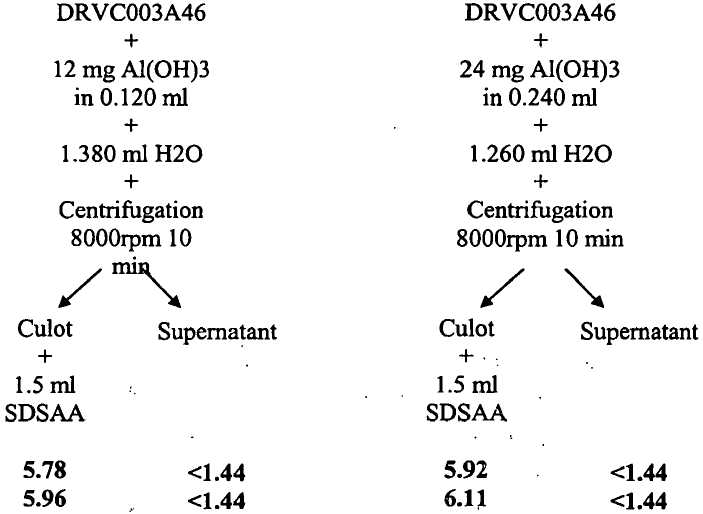 SDSAA= Sucrose 2%, Dextran 4%, Sorbitol 3%, Amino-Acid 2%.

According to the viral titration carried out on the supernatant, the quantity of Al(OH)₃ needed to adsorb Rotavirus seems to be low (starting with one lyophilised dose 5.7 log) scaling Up viral titration):

**Table 10**

| **Al(OH)3** | **Adsorption time** | **Titer in supernatant** |
|---|---|---|
| | | |
| 12 mq | 1 hour RT | 2.7 |
| 24 mg | 1 hour RT | 3.4 |
| 48 mq | 1 hour RT | 3.4 |
| 72 mg | 1 hour RT | 2.0 |
| 96 mg | 1 hour RT | Below detection |
| | | |
| 12 mg | Overnight | 2.7 |
| 24 mg | Overnight | Below detection |
| 48 mg | Overnight | 2.5 |
| | | |
| 12 mg | Immediate | Below detection |
| 24 mg | Immediate | 2.0 |
| 48 mg | Immediate | Below detection |

Time needed to adsorb Rotavirus on Al(OH)₃ seems to be short:
One dose of lyophilised Rotavirus was reconstituted in presence of 24 mg Al(OH)₃, and centrifuged after 0, 15, 60 min and 24 hours. The "culot" were resuspended in SDSAA before viral titration:

**Table 11**

| **Time** | **Culot** | **Supernatant** |
|---|---|---|
| 0 min | 5.26 | 3.17 |
| 15 min | 5.34 | <1.44 |
| 60 min | 5.96 | <1.44 |
| 24 hours | 6.13 | <1.44 |

### 5.8: Using CaCO₃ as antacid

In order to avoid aluminium in the vaccine, the antacid Al(OH)₃ was replaced by another insoluble inorganic salt: CaCO₃ (calcium carbonate).
The phenomena observed with CaCO₃ are parallel to those described for Al(OH)₃:
- Association of Rotavirus with the inorganic salt;
- Maintainance of Rotavirus activity when associated with the inorganic salt;
- Possibility of liberation of Rotavirus from the association by dissolution of the inorganic base by an acid;
- Possibility of co-lyophilisation of the antacid and the Rotavirus.

### CaCO₃ and Rotavirus association

In a first trial, lyophilised Rotavirus (viral titer 5.7) was reconstituted with a suspension of CaCO₃ in water (50mg in 1.5ml); and then centrifuged, and the viral titer of the supernatant compared to the pellet.

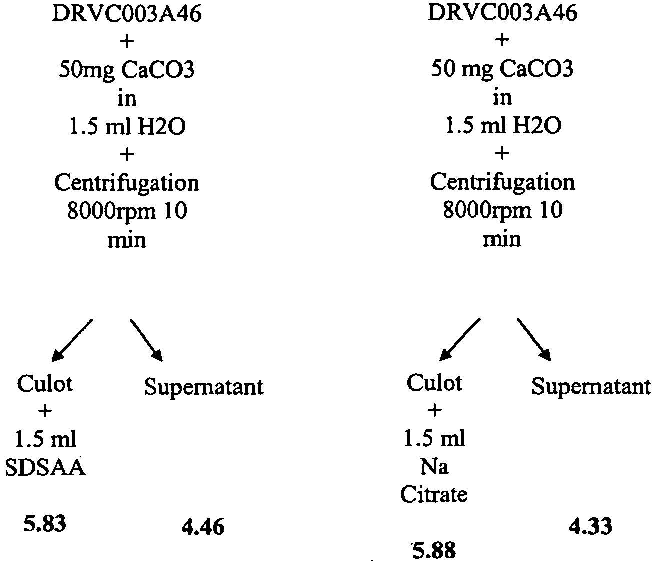

This indicates that more that 90% of the Rotavirus is associated with CaCO₃.

Also, when the virus was associated, it was possible to realise the titration and to recover the original viral quantities.
Also, viral titers are slightly higher that those obtained without CaCO₃.

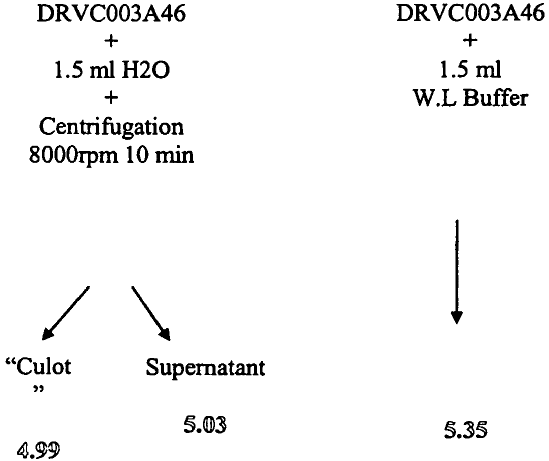

### Quantity of CaC03 and Rotavirus association

Lyophilised Rotavirus was reconstituted with a CaCO₃ suspension in water (1.5ml):
10 mg
50 mg
100 mg
and then centrifuged, and the viral titer of the supernatant compared to the culot.

**Table 12**

| **CaCO3** | **Extempo + Centri.** | | **1 Hour + Centri** | |
|---|---|---|---|---|
| | **Culots** | **Surpernatant** | **Culots** | **Surpernatant** |
| 100mg | 4.57 | 3.01 | 4.79 | 3.09 |
| 50mg | 4.17 | 4.15 | 4.22 | 3.86 |
| 10mg | 3.17 | 4.77 | 3.87 | 4.87 |

Thus, clearly, more CaCO₃ and more virus is associated, and less is found in the supernatant.
However, the full dose is not completely recovered (expected a total of 5.3 at least or even 5.8 as obtained earlier - see above).

### CaCO₃ Protection of Rotavirus during Baby Rossett-Rice antacid titration

Using 10 doses of lyophilised Rotavirus (DRVC003A46) and 50mg of CaCO₃, two types of baby Rossett-Rice titration were carried out:
In a classic Rossett-Rice titration, the antacid is mixed with Rotavirus and HCl is poured into this medium.

In the "inverse" baby Rossett-Rice, the situation is the reverse: antacid is dropped into the HCl pool (as it occurs in vivo).

**Table 13**

| **Classical baby Rossett-Rice titration** | | | |
|---|---|---|---|
| Lyophi. Rota stored at: | Buffer | Theoretical Viral Titer | Measured Viral Titer |
| 4°C | 60 mg CaCO3 | 5.3 | 4.6 |
| -80°C | 60 mg CaCO3 | 5.3 | 4.6 |
| 4°C | 24 mg Al(OH)3 | 5.4 | <2.9 |
| -80°C | 24 mg Al(OH)3 | 5.4 | <2.9 |
| | | | |

| **Inverse baby Rossett-Rice titration** | | | |
|---|---|---|---|
| Lyophi. Rota stored at: | Buffer | Theoretical Viral Titer | Measured Viral Titer |
| 4°C | 60 mg CaCO3 | 5.3 | 4.6 |
| -80°C | 60 mg CaCO3 | 5.3 | 4.6 |
| 4°C | 24 mg Al(OH)3 | 5.4 | <2.9 |
| -80°C | 24 mg Al(OH)3 | 5.4 | <2.9 |

Thus, in this *in vitro* experiment, calcium carbonate is able to protect about 20% of Rotavirus from the presence of HCl, while aluminium hydroxide is not able to.

### 5.9: Lyophilisation of Rotavirus in presence of CaCO₃ antacid:

**Table 14**

| Batch n° | Composition | Viral titer at time = zero after lyophilisation | Viral titer after lyopjhilisation and 1 week at 37°C |
|---|---|---|---|
| 99K08/01 | Sucrose: 2% | 10^{5.28} | 10^{5.10} |
| | Dextran: 4% | | |
| | Sorbitol: 3% | | |
| | Am. Acids: 2% | | |
| | CaCO₃: 50 mg | | |
| 99K08/02 | Sucrose: 2% | 10^{5.16} | 10^{5.15} |
| | Dextran: 4% | | |
| | Sorbitol: 3% | | |
| | Am. Acids: 2% | | |
| | CaCO₃: 60 mg | | |
| 00C24/01 | Sucrose: 2% | 10^{5.07} | 10^{4.69} |
| | Dextran: 4% | | |
| | Sorbitol: 3% | | |
| | Am. Acids: 2% | | |
| | CaCO₃: 60 mg | | |
| | Xanthane 0.3% | | |
| 00C24/03 | Sucrose: 2% | 10^{5.07} | 10^{4.85} |
| | Dextran: 4% | | |
| | Sorbitol: 3% | | |
| | Am. Acids: 2% | | |
| | CaCO₃: 60 mg | | |
| | Xanthane 0.3% | | |
| 00E09/25 | Sucrose: 2% | 10^{5.03} | 10^{4.91} |
| | Dextran: 4% | | |
| | Sorbitol: 3% | | |
| | Am. Acids: 2% | | |
| | CaCO₃: 60 mg | | |
| | Xanthane 0.25% | | |
| 00E09/30 | Sucrose: 2% | 10^{5.01} | 10^{4.87} |
| | Dextran: 4% | | |
| | Sorbitol: 3% | | |
| | Am. Acids: 2% | | |
| | CaCO₃: 60 mg | | |
| | Xanthane 0.30% | | |
| 00F26/06 | Sucrose: 2% | 10^{4.50} | 10^{4.70} |
| | Dextran: 4% | | |
| | Sorbitol: 3% | | |
| | Am. Acids: 2% | | |
| | CaCO₃: 60 mg | | |
| | Starch: 2% | | |

This is the "all in one" - lyophilisation of Rotavirus and antacid (CaCO3) together in the same vial. To prevent sedimentation of CaCO₃ during the filling step, viscous agents are needed. Examples of such viscous agents include Xanthane gum and Starch. The Rotavirus activity is maintained even in the presence of Xanthane gum and Starch.

### 5.10 Lyophilised tablets for quick disintegration when placed in the mouth:

The following formulations demonstrate the "lyoc" concept. That is, quick dissolution of the lyophilised cake in the mouth.

**Table 15**

| Batch n° | Formulation composition | Viral titer before lyophilisation | Viral titer after lyopjhilisation and 1 week at 37° |
|---|---|---|---|
| 99B10/06 | Sucrose 4% | 10^{5.11} | 10^{4.53} |
| | Sodium glutamate 3.7% | | |
| | Al(OH)3 48mg | | |
| 99C11/12 | Maltitol 3% | 10^{4.16} | 10^{3.79} |
| | AI(OH) 48mg | | |
| | Hydroxypropylmethyl-cellulose: 1% | | |

| Batch n° | Fomulation composition | Viral titer at time = zero after lyophilisation | Viral titer after lyopjhilisation and 1 week at 37° |
|---|---|---|---|
| 00C24/05 | Sucrose: 2% | 10^{5.02} | 10^{4.54} |
| | Dextran: 4% | | |
| | Sorbitol: 3% | | |
| | Am. Acids: 2% | | |
| | CaCO₃: 60 mg | | |
| | Xanthane 0.3% | | |
| 00C24/06 | Sucrose: 2% | 10^{4.86} | 10^{4.56} |
| | Dextran: 4% | | |
| | Sorbitol: 3% | | |
| | Am. Acids: 2% | | |
| | CaCO₃: 60 mg | | |
| | Xanthane 0.3% | | |
| 00F26/11 | Sucrose: 1% | 10^{4.70} | 10^{4.40} |
| | Dextran: 2% | | |
| | Sorbitol: 1.5% | | |
| | Am. Acids: 1% | | |
| | CaCO₃: 60 mg | | |
| | Starch: 2% | | |

In the "lyoc concept" both Xanthane and Starch can be used (maintaining the quick dissolution properties of the lyophilised cake).

### Example 6: Use of Calcium Carbonate as the antacid for the Rotavirus vaccine composition

When a suspension of CaCO₃ in water is used as the antacid for Rotavirus there is a problem that the calcium carbonate particles sediment rapidly when placed in water since the powder density value approaches 2.6 and the average particle size is 30µm. This sedimentation can be slowed by:
1 increasing the density of the surrounding medium
2 increasing the viscosity of the surrounding medium
3 reducing the particles size
4 keeping particles away from each other

### 6.1: Increasing density of the surrounding medium:

When the CaCO₃-Water suspension (when placed in the syringe) is placed on the lyophilised cake (containing sucrose 2%, dextran 4%; sorbitol 3%; amino-acids 2%) the density of the surrounding medium is increased, but the speed of CaCO₃ sedimentation is not very much different from the CaCO₃-Water suspension.

### 6.2 Increasing the viscosity of the surrounding medium:

### Pseudoplastic excipents

A pseudoplastic solution is defined as a solution having higher viscosity on standing compared to its viscosity under agitation.
Usual excipients of this type are:
natural Polymers for example:
   arabic gum
   adragante gum
   agar-agar
   alginates
   pectines
semi-synthetic polymers for example:
   carboxymethylcellulose (Tyloses C®)
   methylcellulose (Methocels A®, Viscotrans MC®, Tylose MH® and MB®) hydroxypropylcellulose (Klucels®)
   hydroxypropylmethylcellulose (Methocels E® and K®, Viscontrans MPHC®)

In general those pseudoplastic excipients are used together with thixotropic agents.

### Pseudoplastic excipients with low flowing capacity

Those polymers, at a sufficient concentration, give rise to a structural fluid arrangement resulting in a high viscosity solution having low flowing capacity on standing. A certain quantity of energy needs to be given to the system to allow flowing and transfer. External energies (agitation) are needed to destroy temporarily the structural fluid arrangement in order to obtain a fluid solution.
Examples of such polymers are Carbopols® and Xanthane gum.

### Thixotropic excipents

With these excipents, on standing, a gel structure is obtained; while under agitation a fluid solution is obtained.

Examples of thixotropic excipients are: Veegum ®(Magnesium-aluminium silicate) and Avicel RC® (about 89% microcrystalline cellulose and 11% Caboxymethylcellulose Na).

### 6.3 Reducing the particles size

A reduction in the CaCO₃ particle size resulted in a decrease in the antacid capacity of the compound.

### 6.4 Keeping particles away from each other

This is the case in Veegum® and Avicel® for which insoluble particles smaller (about 1 µm) than the CaCO₃ particles, are placed between CaCO₃ particles in order to prevent aggregation.

### Example 7: Product design

The following schemes demonstrate examples of possible product designs.

### 7.1 CaCO₃ in the syringe

Having already clinical batches of Rotavirus in lyophilised vials, the antacid can be placed in the reconstituent liquid contained in the syringe.

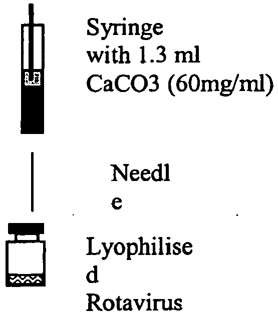

In this product presentation, sedimentation of CaCO₃ must be under control not only during the filling steps, but also during the complete shelf-live of the product (at least 2 years).

### 7. 2 CaCO₃ in the lyophilised vial

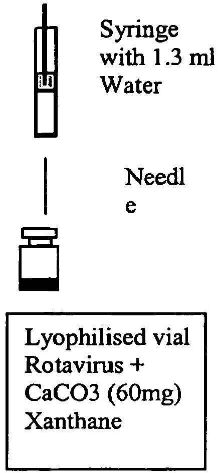

### 7.3. Lyophilisation in a blister

In this case Rotavirus, CaCO₃ and Xanthane gum are lyophilised together directly in the blister.

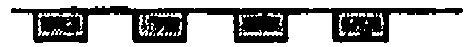

### Example 8: Lyophilisation of different strain of Rotavirus

**Table 16**

| Batch n° | Rotavirus strain | Fomulation composition | Viral titer at t = zero after lyophilisation | Viral titer after lyopjhilisation and 1 week at 37° |
|---|---|---|---|---|
| 00F26/01 | G1 | Sucrose: 2% | 10^{4.6} | 10^{4.7} |
| | SB purif n°61 | Dextran: 4% | | |
| | PR0/0232 | Sorbitol: 3% | | |
| | | Am. Acids: 2% | | |
| 00F26/02 | G2 (DS-1) | Sucrose: 2% | 10^{4.4} | 10^{4.4} |
| | | Dextran: 4% | | |
| | | Sorbitol: 3% | | |
| | | Am. Acids: 2% | | |
| 00F26/03 | G3(P) | Sucrose: 2% | 10^{4.6} | 10^{4.5} |
| | | Dextran: 4% | | |
| | | Sorbitol: 3% | | |
| | | Am. Acids: 2% | | |
| 00F26/04 | G4 (VA-70) | Sucrose: 2% | 10^{4.8} | 10^{4.8} |
| | | Dextran: 4% | | |
| | | Sorbitol: 3% | | |
| | | Am. Acids: 2% | | |
| 00F26/05 | G9(W161) | Sucrose: 2% | 10^{4.6} | 10^{4.5} |
| | | Dextran: 4% | | |
| | | Sorbitol: 3% | | |
| | | Am. Acids: 2% | | |

The strains DS-1, P and VA70 are described as Human rotavirus reference strains for serotype G2, G3 and G4 respectively at page 1361 of "Fields" Raven press 1990, second edition.

In this experiment different Rotavirus strains have been lyophilised.
For all, both the viral titer have been maintained during lyophilisation and accelarated stability (one week at 37°C) has been shown.

### Example 9: Phase I safety study in adults of one oral administration of the Rotavirus vaccine.

A Phase I study was carried out to assess the safety and reactogenicity of a single oral dose of 10^{6.0} ffu of the P43 vaccine in healthy adults aged 18 to 45 years.

The clinical trial was double blind and randomized. It was placebo-controlled and self-contained. The study was performed in one single centre in Belgium.

### Study Population

A total of 33 subjects, 11 in the placebo group and 22 in the vaccine group, were enrolled and all completed the study. All volunteers were Caucasians. Their mean age at the time of vaccination was 35.3 years, with a range of 18 to 44 years. The trial began in January and ran for just over one month.

### Material

### Vaccine

Clinical lots of P43 vaccine were produced, purified, formulated and lyophilized according to Good Manufacturing Practices. The lots were released by Quality Control and Quality Assurance. Each vial of vaccine contained the following components:
Active ingredient:

| | |
|---|---|
| P43 strain | Min. 10^{5.8} ffu |

Excipients, stabilizers:

| | |
|---|---|
| Sucrose | 9 mg |
| Dextran | 18 mg |
| Sorbitol | 13.5 mg |
| Amino acids | 9 mg |

### Placebo

Vials of placebo were prepared and released. Each vial of placebo contained the following components:

### Excipients, stabilizers:

| | |
|---|---|
| Sucrose | 9 mg |
| Dextran | 18 mg |
| Sorbitol | 13.5 mg |
| Amino acids | 9 mg |

### Diluent

Water for injection was used as diluent to reconstitute vaccine and placebo.

### Administration

Approximately 10 to 15 minutes before administration of the vaccine or the placebo, subjects of both groups were given 10 ml of Mylanta® orally. Mylanta® is a registered antacid. The antacid increases the pH of the stomach and prevents inactivation of the rotavirus during its passage through the stomach.

To prepare the vaccine, two vials of lyophilized P43 containing 10^{5.8} ffu per vial were reconstituted with 1.5 ml of diluent water for injection. This achieved a calculated viral titer of 10^{6.1} ffu per dose. The reconstituted vaccine was administered promptly as a single oral dose.

To prepare the placebo, two vials of lyophilized placebo were reconstituted with 1.5 ml water for injection and administered orally as a single dose.

### Safety and Reactogenicity

The following criteria of safety and reactogenicity applied:
Solicited general symptoms were fever, diarrhea, vomiting, nausea, abdominal pain and loss of appetite. They were recorded during eight days post administration.
Unsolicited symptoms were recorded during 30 days post administration.
Serious adverse events were recorded during the entire study period.
Diarrhea samples were to be collected during eight days post administration.

The results were:
No solicited symptoms, no unsolicited and no serious adverse events were reported during the respective observation periods.
No cases of diarrhea were reported.

### Conclusions

SB Biologicals P43 vaccine was safe relative to the placebo when administered orally in a double-blind fashion as a single dose at the dose of 10^{6.1} ffu to healthy adult volunteers aged 18 to 44.

### Example 10 - Efficacy of two doses of a human monovalent Rotavirus vaccine, Rotarix™ in preventing Gastro-enteritis due to G1 and non-G1 (G9) Rotavirus

A randomised, double-blind, placebo-controlled phase II trial was conducted to evaluate the protective efficacy of a vaccine derived from the G1P human strain 89-12 for infant immunisation.

Specifically the vaccine used was named Rotarix™ and comprises as the rotavirus component the attenuated G1 human strain deposited as ECACC deposit 99081301.

493 healthy infants received two doses of Rotarix™ at the viral concentration (10⁶ ffu) or placebo (504) at age 2 and 4 months, concomitantly with DTPw-HBV and Hib vaccines, OPV was administered 2 weeks apart. Diarrhoeal samples were tested for the presence of rotavirus (ELISA) and the serotypes determined in positive samples (RT-PCR). Diarrhoeal episodes reported from two weeks after the second dose were considered for the efficacy analysis. Severity was determined using a 20-point scale (Ruuska and Vesikari, 1990). A score ≥ 11 defined severe disease.

**Results**. An interim analysis of efficacy was performed on the above mentioned group and the isolated serotypes were G1 and G9, almost evenly distributed. The overall attack rate in the placebo group varied from 4.8% for G1 to 3.6% for G9 during the 6 months observation period. Two doses of Rotarix™ at 10⁶ ffu protected against all types of diarrhoea caused by G1 with 83% efficacy [95% Cl: 50.4-95.7] and 92 % efficacy [95% Cl: 47.6-99.8] against severe gastro-enteritis. If the diarrhoea was caused by G9, the protection against all types of diarrhoea was 60% [95% Cl: 0.2-86.0] and 81% [95% Cl: 33.0-96.4] against severe gastro-enteritis. For each of these efficacy endpoints, there was a statistically significant decrease in diarrhoea episodes in the HRV group as compared to the placebo group (p < 0.05, two-sided Fisher's exact test).

**Conclusion**. These results are highly supportive of the efficacy of 2 doses of a monovalent HRV vaccine, Rotarix™, in protecting young infants against the homologous G1 strain and cross-protect against the G9 strain.

### Example 11 - Efficacy of two doses of a human monovalent Rotavirus vaccine: Rotarix™ administered at three different virus concentrations in preventing Gastro-enteritis due to G1 and non-G1 (G2, G3, G4, G9) Rotavirus

A randomised, double-blind, placebo-controlled phase II trial was conducted to evaluate the protective efficacy and efficacy against hospitalization of a vaccine derived from the G1P human strain 89-12 for infant immunisation.

Specifically the vaccine used was named Rotarix™ and comprises as the rotavirus component the attenuated G1 human strain deposited as ECACC deposit 99081301.

Healthy infants received two doses of Rotarix™ at three different virus concentrations (468 received 10^{4.7} ffu, 460 received 10^{5.2} ffu; 464 received 10^{5.8} ffu) or placebo (454) at age 2 and 4 months, concomitantly with DTPw-HBV and Hib vaccines, OPV was administered 2 weeks apart. Diarrhoeal samples were tested for the presence of rotavirus (ELISA) and the serotypes determined in positive samples (RT-PCR). Diarrhoeal episodes reported from two weeks after the second dose until subjects were one year of age were considered for the efficacy analysis. Severity was determined using a 20-point scale (Ruuska and Vesikari, 1990). A score ≥ 11 defined severe disease.

### Results:

Results are illustrated in the tables below. Infants in the vaccine groups had significantly fewer rotavirus gastroenteritis episodes than children in the placebo group (p < 0.001, two-sided Fisher's exact test). Depending on the dosage, protective efficacy against severe rotavirus gastroenteritis reached 86% (95%Cl: 63%-96%), and 70% (95%Cl, 46%-84%) against any rotavirus gastroenteritis. For each of these efficacy endpoints, there was a statistically significant decrease in diarrhoea episodes in the HRV group as compared to the placebo group (p < 0.001, two-sided Fisher's exact test). Multiple rotavirus serotypes (G1, G2, G3, G4 and G9) were identified from gastroenteritis stools (ELISA and RT-PCR) allowing to also calculate vaccine efficacy against non-G1 serotypes. As can be seen from table 19 in particular, for non-G1 serotypes (G2, G3, G4 and G9), and depending on the dosage, efficacy against severe rotavirus gastroenteritis reached 83% (95%Cl: 40%-97%), providing proof of concept that the monovalent G1-based G1P1A P[8] human rotavirus vaccine elicits cross-protection against heterotypic (i.e. non-G1) strains.

### Features of rotavirus gastro-enteritis episodes reported during the study

**Table 17**

| | | **RIX441410^{4.7} ffu** | **RIX4414 10^{5.2} ffu** | **RIX4414 10^{5.8} ffu** | **Placebo** |
|---|---|---|---|---|---|
| Any rotavirus gastroenteritis | | 21 | 22 | 15 | 51 |

| **no. of episodes (percent) with specific feature among all rotavirus gastroenteritis episodes reported** | | | | | |
|---|---|---|---|---|---|
| **Severity scores** | <7 | 4 (19) | 8 (36) | 2 (13) | 5 (10) |
| | 7-10 | 5(24) | 4 (18) | 8 (53) | 12 (24) |
| | ≥ 11 | 12 (57) | 10 (45) | 5 (33) | 34 (67) |
| **Identified rotavirus serotypes** | | | | | |
| | | | | | |
| | wild G1 | 12 (57) | 6 (27) | 7 (47) | 30 (59) |
| | G2 | 0 | 0 | 1 (7) | 3 (6) |
| | G3 | 1 (5) | 0 | 0 | 2 (4) |
| | G4 | 0 | 0 | 1 (7) | 0 |
| | G9 | 8 (38) | 14 (64) | 7 (47) | 15 (29) |
| | Canine | 0 | 0 | 0 | 1(2) |
| | Unknown | 0 | 2 (9) | 0 | 0 |

### Protective efficacy of two doses of RIX4414 human rotavirus vaccine against rotavirus gastroenteritis

**Table 18**

| | | **Any rotavirus gastroenteritis** | | **Severe rotavirus gastroenteritis** | | **Hospitalization for rotavirus gastroenteritis** | |
|---|---|---|---|---|---|---|---|
| | **N** | **n(%)** | **Efficacy (95% Cl)** | **n(%)** | **Efficacy (95% Cl)** | **n(%)** | **Efficacy (95% Cl)** |
| Pooled vaccine groups | 1392 | 58 (4)* | 61 (42-74) | 27 (2)* | 74 (56-85) | 9 (0.6) * | 79 (48-92) |
| RIX4414 10^{5.8} ffu | 464 | 15 (3)* | 70 (46-84) | 5 (1)* | 86(63-96) | 3 (0.6)‡ | 79(25-96) |
| RIX4414 10^{5.2} ffu | 460 | 22 (5)* | 56 (25-75) | 10 (2)* | 71 (40-87) | 1 (0.2)* | 93 (54-100) |
| RIX4414 10^{4.7} ffu | 468 | 21 (4)* | 58 (29-76) | 12 (3)* | 66 (32-84) | 5 (1)† | 65(-2-90) |
| Placebo | 454 | 49(11) | - | 34 (7) | - | 14 (3) | - |

| | | | | | | | |
|---|---|---|---|---|---|---|---|
| *p<0.001 for each comparison between the vaccine and placebo groups by two-sided Fisher's exact test (significant level of α=0.05) †p=0.037 for the comparison between the vaccine and placebo groups by two-sided Fisher's exact test (significant level of α=0.05) ‡p=0.007 for the comparison between the vaccine and placebo groups by two-sided Fisher's exact test (significant level of α=0.05) N = number of subjects n/% = number of subjects reporting at least one specified rotavirus gastroenteritis episode Exact 95% confidence intervals are shown | | | | | | | |

### Protective efficacy of two doses of RIX4414 human rotavirus vaccine against serotype specific severe rotavirus gastroenteritis

**Table 19**

| | | **Severe rotavirus gastroenteritis** | | |
|---|---|---|---|---|
| | **N** | **n(%)** | **Efficacy (95% Cl)** | **p-value*** |
| **G1 wild type rotavirus** | | | | |
| Pooled vaccine groups | 1392 | 13 (0.9) | 74 (41- 88) | <0.001 |
| RIX4414 10^{5.8} ffu | 464 | 2 (0.4) | 88 (48-99) | <0.001 |
| RIX4414 10^{5.2} ffu | 460 | 4(0.9) | 75 (24-94) | 0.006 |
| RIX4414 10^{4.7} ffu | 468 | 7(1.5) | 58 (-9-85) | 0.057 |
| Placebo | 454 | 16 (4) | - | - |

| **Non-G1 rotavirus (mainly G9 with G2, G3 and G4 types)** | | | | |
|---|---|---|---|---|
| Pooled vaccine groups | 1392 | 14 (1) | 73 (42-88) | <0.001 |
| RIX4414 10^{5.8} ffu | 464 | 3(0.6) | 83 (40-97) | 0.001 |
| RIX4414 10^{5.2} ffu | 460 | 6 (1) | 65 (7-89) | 0.020 |
| RIX4414 10^{4.7} ffu | 468 | 5 (1) | 71 (19-92) | 0.009 |
| Placebo | 454 | 17 (4) | - | - |

| | | | | |
|---|---|---|---|---|
| *Two-sided Fisher's exact test (significant level of α=0.05) used.for each comparison between the vaccine and placebo groups. N = number of subjects n/% = number of subjects reporting at least one specified rotavirus gastroenteritis episode Exact 95% confidence intervals are shown | | | | |

### Conclusion:

These results are highly supportive of the efficacy of 2 doses of a monovalent HRV vaccine, Rotarix™, in protecting young infants against any and severe rotavirus gastroenteritis caused by the homologous G1 strain and broad cross-protection against heterologous strains, namely G2, G3, G4 and G9.

## Claims

1. Use of an attenuated human rotavirus strain from a G1 serotype in the preparation of a composition for inducing an immune response against rotavirus infection from the G1 and at least two of the non-G1 rotavirus serotypes selected from the group consisting of: G2, G3, G4 and G9 serotypes, wherein the attenuated rotavirus strain is a single rotavirus variant, or substantially a single rotavirus variant, having a VP4 gene comprising, in the nucleotide sequence, an adenine (A) at positions 788 and 802 and a thymine (T) at position 501 from the start codon; and wherein the VP7 gene comprises, in the nucleotide sequence, a thymine (T) at position 605 and an adenine (A) at position 897 from the start codon.

2. Use of an attenuated rotavirus strain from a G1 serotype in the preparation of a composition for inducing an immune response against rotavirus infection from the G1 and at least two of the non-G1 rotavirus serotypes selected from the group consisting of: G2, G3, G4 and G9 serotypes, wherein the attenuated rotavirus strain is ECACC deposit 99081301, or a single rotavirus variant, or substantially a single rotavirus variant, obtainable or derivable from ECACC deposit 99081301 and having a VP4 gene comprising, in the nucleotide sequence, an adenine (A) at positions 788 and 802 and a thymine (T) at position 501 from the start codon; and wherein the VP7 gene comprises, in the nucleotide sequence, a thymine (T) at position 605 and an adenine (A) at position 897 from the start codon .

3. Use according to claim 1 or claim 2, wherein the composition is capable of inducing an immune response against rotavirus infection from at least three of the non-G1 rotavirus serotypes selected from the group consisting of: G2, G3, G4 and G9 serotypes.

4. Use according to claim 1 to 3, wherein the composition is capable of inducing an immune response to the G1 and to the G2, G3, G4 and G9 serotypes.

5. Use according to claims 1 to 4 wherein the composition is at least 50% protective, in a population of vaccinated individuals, against diarrhea and/or gastroenteritis and/or severe gastroenteritis caused by infection of a rotavirus of at least two non-G1 serotypes.

6. Use according to claims 1 to 4 wherein the composition is up to 60% protective, in a population of vaccinated individuals, against diarrhoea caused by infection of a rotavirus of at least two non-G1 serotypes.

7. Use according to claims 1 to 4 wherein the composition is up to 81% protective against gastro-enteritis caused by infection of a rotavirus of at least two non-G1 serotypes.

8. Use according to claims 1 to 4 wherein the composition is up to 83% protective in a population of vaccinated individuals against severe gastro-enteritis caused by infection of rotaviruses of at least two non-G1 serotypes.

9. Use according to claim 5, 7 and 8 wherein the gastro-enteritis is caused by infection of a rotavirus of at least three non-G1 serotypes selected from the group consisting of G2, G3, G4 and G9.

10. Use according to claim 9 wherein the gastro-enteritis is caused by infection with G2, G3, G4 and G9 serotypes.

11. Use according to claims 1 to 10, wherein the composition is for administration in a 2-dose regime.

12. Use according to claims 1 to 11 wherein the attenuated rotavirus strain is formulated with a suitable pharmaceutical carrier or with an antacid buffer or both.

## Patentansprüche

1. Verwendung eines attenuierten humanen Rotavirus-Stammes vom G1-Serotyp in der Herstellung einer Zusammensetzung zur Induktion einer Immunantwort gegen Rotavirus-Infektion durch den G1- und mindestens zwei der Nicht-G1-Rotavirus-Serotypen, die ausgewählt sind aus der Gruppe bestehend aus: G2-, G3-, G4- und G9-Serotypen, wobei der attenuierte Rotavirus-Stamm eine einzelne Rotavirus-Variante oder im wesentlichen eine einzelne Rotavirus-Variante mit einem VP4-Gen ist, das in der Nukleotidsequenz ein Adenin (A) an den Positionen 788 und 802 und ein Thymin (T) an Position 501 ab dem Start-Kodon umfasst; und wobei das VP7-Gen in der Nukleotidsequenz ein Thymin (T) an Position 605 und ein Adenin (A) an Position 897 ab dem Start-Kodon umfasst.

2. Verwendung eines attenuierten Rotavirus-Stammes vom G1-Serotyp in der Herstellung einer Zusammensetzung zur Induktion einer Immunantwort gegen Rotavirus-Infektion durch den G1- und mindestens zwei der Nicht-G1-Rotavirus-Serotypen, die ausgewählt sind aus der Gruppe bestehend aus: G2-, G3-, G4- und G9-Serotypen, wobei der attenuierte Rotavirus-Stamm die ECACC-Hinterlegung 99081301 oder eine einzelne Rotavirus-Variante oder im wesentlichen eine einzelne Rotavirus-Variante ist, erhältlich oder ableitbar von der ECACC-Hinterlegung 99081301 und mit einem VP4-Gen, das in der Nukleotidsequenz ein Adenin (A) an den Positionen 788 und 802 und ein Thymin (T) an Position 501 ab dem Start-Kodon umfasst; und wobei das VP7-Gen in der Nukleotidsequenz ein Thymin (T) an Position 605 und ein Adenin (A) an Position 897 ab dem Start-Kodon umfasst.

3. Verwendung gemäß Anspruch 1 oder Anspruch 2, wobei die Zusammensetzung imstande ist, eine Immunantwort gegen Rotavirus-Infektion durch mindestens drei der Nicht-G1-Rotavirus-Serotypen zu induzieren, die ausgewählt sind aus der Gruppe bestehend aus: G2-, G3-, G4- und G9-Serotypen.

4. Verwendung gemäß Anspruch 1 bis 3, wobei die Zusammensetzung imstande ist, eine Immunantwort gegen den G1- und gegen die G2-, G3-, G4- und G9-Serotypen zu induzieren.

5. Verwendung gemäß den Ansprüchen 1 bis 4, wobei die Zusammensetzung in einer Population geimpfter Individuen zu mindestens 50 % Schutz bietet gegen Durchfall und/oder Gastroenteritis und/oder schwere Gastroenteritis, verursacht durch Infektion eines Rotavirus von mindestens zwei Nicht-G1-Serotypen.

6. Verwendung gemäß den Ansprüchen 1 bis 4, wobei die Zusammensetzung in einer Population geimpfter Individuen bis zu 60 % Schutz bietet gegen Durchfall, verursacht durch Infektion eines Rotavirus von mindestens zwei Nicht-G1-Serotypen.

7. Verwendung gemäß den Ansprüchen 1 bis 4, wobei die Zusammensetzung bis zu 81 % Schutz bietet gegen Gastroenteritis, verursacht durch Infektion eines Rotavirus von mindestens zwei Nicht-G1-Serotypen.

8. Verwendung gemäß den Ansprüchen 1 bis 4, wobei die Zusammensetzung in einer Population geimpfter Individuen bis zu 83 % Schutz bietet gegen schwere Gastroenteritis, verursacht durch Infektion von Rotaviren von mindestens zwei Nicht-G1-Serotypen.

9. Verwendung gemäß Anspruch 5, 7 und 8, wobei die Gastroenteritis durch Infektion eines Rotavirus von mindestens drei Nicht-G1-Serotypen verursacht wird, die ausgewählt sind aus der Gruppe bestehend aus G2, G3, G4 und G9.

10. Verwendung gemäß Anspruch 9, wobei die Gastroenteritis durch Infektion mit G2-, G3-, G4- und G9-Serotypen verursacht wird.

11. Verwendung gemäß den Ansprüchen 1 bis 10, wobei die Zusammensetzung für die Verabreichung in einem 2-Dosis-Schema bestimmt ist.

12. Verwendung gemäß den Ansprüchen 1 bis 11, wobei der attenuierte Rotavirus-Stamm mit einem geeigneten pharmazeutischen Träger oder mit einem antaziden Puffer oder beidem formuliert ist.

## Revendications

1. Utilisation d'une souche atténuée de rotavirus humain d'un sérotype G1 dans la préparation d'une composition destinée à l'induction d'une réponse immunitaire contre une infection à rotavirus du sérotype G1 et d'au moins deux sérotypes non G1 de rotavirus choisis dans le groupe constitué par : les sérotypes G2, G3, G4 et G9, où la souche atténuée de rotavirus est un variant unique de rotavirus, ou pratiquement un variant unique de rotavirus, comportant un gène VP4 comprenant, dans la séquence nucléotidique, une adénine (A) au niveau des positions 788 et 802 et une thymine (T) au niveau de la position 501 à partir du codon de départ ; et où le gène VP7 comprend, dans la séquence nucléotidique, une thymine (T) au niveau de la position 605 et une adénine (A) au niveau de la position 897 à partir du codon de départ.

2. Utilisation d'une souche atténuée de rotavirus d'un sérotype G1 dans la préparation d'une composition destinée à l'induction d'une réponse immunitaire contre une infection à rotavirus du sérotype G1 et d'au moins deux sérotypes non G1 de rotavirus choisis dans le groupe constitué par : les sérotypes G2, G3, G4 et G9, où la souche atténuée de rotavirus est le dépôt ECACC 99081301 ou un variant unique de rotavirus, ou pratiquement un variant unique de rotavirus, pouvant être obtenu ou dérivé du dépôt ECACC 99081301 et comportant un gène VP4 comprenant, dans la séquence nucléotidique, une adénine (A) au niveau des positions 788 et 802 et une thymine (T) au niveau de la position 501 à partir du codon de départ ; et où le gène VP7 comprend, dans la séquence nucléotidique, une thymine (T) au niveau de la position 605 et une adénine (A) au niveau de la position 897 à partir du codon de départ.

3. Utilisation selon la revendication 1 ou la revendication 2, où la composition est capable d'induire une réponse immunitaire contre une infection à rotavirus d'au moins trois sérotypes non G1 de rotavirus choisis dans le groupe constitué par : les sérotypes G2, G3, G4 et G9.

4. Utilisation selon les revendications 1 à 3, où la composition est capable d'induire une réponse immunitaire contre le sérotype G1 et contre les sérotypes G2, G3, G4 et G9.

5. Utilisation selon les revendications 1 à 4, où la composition est au moins protectrice à 50 %, dans une population d'individus vaccinés, contre la diarrhée et/ou la gastro-entérite et/ou la gastro-entérite sévère provoquée par l'infection d'un rotavirus d'au moins deux sérotypes non G1.

6. Utilisation selon les revendications 1 à 4, où la composition est protectrice jusqu'à 60 %, dans une population d'individus vaccinés, contre la diarrhée provoquée par l'infection d'un rotavirus d'au moins deux sérotypes non G1.

7. Utilisation selon les revendications 1 à 4, où la composition est protectrice jusqu'à 81 % contre la gastro-entérite provoqués par l'infection d'un rotavirus d'au moins deux sérotypes non G1.

8. Utilisation selon les revendications 1 à 4, où la composition est protectrice jusqu'à 83 % dans une population d'individus vaccinés contre une gastro-entérite sévère provoquée par l'infection d'un rotavirus d'au moins deux sérotypes non G1.

9. Utilisation selon les revendications 5, 7 et 8, où la gastro-entérite est provoquée par l'infection d'un rotavirus d'au moins trois sérotypes non G1 choisis dans le groupe constitué de G2, G3, G4 et G9.

10. Utilisation selon la revendication 9, où la gastro-entérite est provoquée par une infection avec des sérotypes G2, G3, G4 et G9.

11. Utilisation selon les revendications 1 à 10, où la composition est destinée à une administration dans un régime à 2 doses.

12. Utilisation selon les revendications 1 à 11, où la souche atténuée de rotavirus est formulée avec un support pharmaceutique approprié ou avec un tampon antiacide ou les deux.
